# FASCICULE DE BREVET EUROPEEN

(11) **EP 3 769 684 B1**
(45) Date de publication et mention de la délivrance du brevet: **18.03.2026**
(21) Numéro de dépôt: 20186695.1
(22) Date de dépôt: 20.07.2020
(51) Int. Cl.: A61B 5/20

(54) **SYSTEME DE DETECTION DE L'INCONTINENCE DE PERSONNES ALITEES**
SYSTEM ZUR INKONTINENZERKENNUNG BEI BETTLÄGERIGEN PERSONEN
SYSTEM FOR DETECTING INCONTINENCE IN BEDRIDDEN PERSONS

(30) Priorité: 23.07.2019 FR 1908357
(43) Date de publication de la demande: 27.01.2021
(73) Titulaire: WINNCARE FRANCE, 85670 Saint-Paul-Mont-Penit (FR)
(72) Inventeur: JOUET, Pascal, 85670 SAINT-PAUL-MONT-PENIT (FR); BARTHELAT, Julien, 85670 SAINT-PAUL-MONT-PENIT (FR); HEBRARD, Jocelyn, 85670 SAINT-PAUL-MONT-PENIT (FR); GEAY, Pierre, 85670 SAINT-PAUL-MONT-PENIT (FR)
(74) Mandataire: Germain Maureau

(56) Documents cités:
- FR-A1- 3 062 207
- FR-A1- 3 067 245
- JP-A- 2016 524 161
- US-A1- 2013 324 955
- US-A1- 2017 112 681
- US-A1- 2017 172 813

## Description

L'invention a trait au domaine des systèmes de détection d'épisode d'incontinence, en particulier de systèmes de détection de liquide émis par des personnes incontinentes alitées.

L'invention trouve une application avantageuse dans les lits, en particulier les lits médicalisés installés en établissement hospitalier, institutions médicalisés, maison de retraite, maison de repos ou domicile.

L'invention est notamment à destination d'individus alités, par exemple des patients atteints de pathologies physiques et/ou mentales, des personnes physiquement diminuées. L'invention vise en particulier les sujets âgés.

L'incontinence urinaire est définie par la Société internationale de la continence *(International continence society)* comme étant toute perte involontaire d'urine par le méat urinaire devenant un problème social ou d'hygiène. Un épisode d'incontinence urinaire est une miction incontrôlée.

L'incontinence fécale est définie comme étant une perte incontrôlée de selles. Un épisode d'incontinence fécale est une défécation incontrôlée.

Le terme incontinence désigne ici indifféremment l'incontinence urinaire ou l'incontinence fécale.

Bien qu'il soit difficile d'établir la prévalence de l'incontinence urinaire dans la population en général, il semble que près de 5% des personnes en soit atteinte (Regat-Bikoï et al., Recherche en soins infirmiers 2013/4 n°115, P.59-67*).* En France, plus de trois millions de personnes souffriraient de l'incontinence (Haab, Rapport sur le thème de l'incontinence urinaire, avril 2007*, la documentation française).*

Le vieillissement et la dépendance physique et/ou mentale sont les principaux facteurs favorisant la survenue d'une incontinence urinaire. Les personnes âgées de plus de 65 ans hospitalisées ou vivant en établissement médicalisés sont les plus concernées, puisque l'incontinence de cette population est estimée entre 49% et 77% *(*Saxers et al. Prevalence and incidence of urinary incontinence of Swiss nursing home residents at admission and after six, 12 and 24 months. Journal of clinical nursing, août 2008*).* Selon le dossier *Solidarité Santé n°22-2011 de la Direction de la Recherche, des Etudes, de l'Evaluation et des Statistiques (DREES),* les personnes âgées en institution, 32% des résidents en EHPA (Etablissement d'Hébergement pour Personnes Âgées) sont atteints d'incontinence urinaire. Par ailleurs, ce même document révèle que la prévalence de l'incontinence augmente avec le niveau de dépendance (mesuré par utilisation du critère d'iso-ressource). Ainsi, près de 63% des personnes appartenant au groupe iso-ressources 1, c'est-à-dire des personnes confinées au lit ou en fauteuil et ayant des facultés intellectuelles gravement altérées, sont atteintes d'incontinence.

L'incontinence fécale touche en France près de 6% de la population en général *(*Denis et al., Prevalence of anal incontinence in adults, Gastroentérologie Clinique et Biologique, 1992, pp. 344-350*),* mais atteint près de 50% en institution *(*Les bonnes pratiques de soins en Etablissement d'Hébergement pour Personnes Âgées Dépendantes, Direction Générale de la Santé, Octobre 2007, p.64).

L'incontinence urinaire peut être associée à une incontinence fécale, l'incontinence est alors dite mixte.

L'incontinence altère considérablement la qualité de vie des personnes, et tout particulièrement pour les personnes alitées.

L'impact est tout d'abord émotif et psychologique. Ainsi, les études prouvent que les personnes souffrant d'incontinence urinaire présentent des symptômes de dépressions, d'anxiété et un sentiment d'insatisfaction plus important que les personnes continentes *(*Ko et AI., The impact of urinary incontinence on quality of life of the elderly, The American Journal Of Managed Care, Vol.11 No.4, Sup, Juillet 2005*).* Les conséquences psychologiques de l'incontinence fécale, bien que moins connues que celles de l'incontinence urinaire, sont essentiellement un retentissement sur l'estime de soi de l'individu incontinent, contribuant à la survenue de réactions émotionnelles d'anxiété, de colère, de mélancolie et de dépression *(*Watier, Incontinence fécale : aspects psychologiques et qualité de vie, Acta Endoscopia, août 2004, volume 34, Issue 4, pp 555-560*).*

L'incontinence urinaire a également des conséquences physiques, telle que des infections urinaires, des habitudes de sommeil perturbées, des risques de chute ou l'atteinte à l'intégrité de la peau *(*Gogniat et al. Incontinence urinaire : connaissances, représentations et pratiques des soignants, Recherche en soins infirmiers 2011/4 N° 107, pp 85-97*, tableau 1).*

L'atteinte à l'intégrité de la peau, qui se matérialise par une dermite associée à l'incontinence (DAI), est une complication fréquente dans les établissements de soin. Une telle inflammation de la peau a longtemps été confondue avec des escarres de stade I ou II. La prévalence de la DAI varie de 5,6 à 50 % et son incidence de 3,4 à 25 % selon la population étudiée et le lieu *(*Faucher et al. Repères en gériatrie, mai 2017, vol.19).

L'apparition de la DAI est causée par une perturbation de la fonction de barrière normale de la peau, provoquée par les composés organiques des fuites, menant à une surhydratation de la peau. Une telle surhydratation de la peau induit sensibilité aux irritations mécaniques (comme les frottements des vêtements, des protections ou de la literie), et réduit la protection tissulaire cutanée à la friction, au cisaillement, ainsi qu'à la pression *(*St-Cyr, Dermite d'incontinence, Perspective infirmière, mars avril 2011, pp 36-40*).* Il a été ainsi mesuré que le risque de développer un ulcère de pression est multiplié par vingt-deux pour un individu adulte hospitalisé atteint d'incontinence fécale, par rapport à un individu adulte non incontinent *(*Maklebust et al. Risk factors associated with having a pressure ulcer: a secondary data analysis, Adv Wound Care. novembre 1994*).*

Par ailleurs, l'incontinence a également pour effet d'augmenter le pH cutané, créant un terrain favorisant la prolifération de microorganismes, provoquant infection mycologiques ou bactériennes.

Pour les personnes alitées, suite à un épisode d'incontinence, il est par conséquent important, tant pour des raisons psychiques que physiques, de procéder à une prise en charge rapide de l'individu incontinent.

Cependant, l'observation d'un épisode d'incontinence peut s'avérer complexe à mettre en œuvre, en particulier en institution.

En effet, les individus concernés peuvent tarder à informer, ou ne pas informer du tout l'équipe soignante. Une telle situation peut toucher des individus qui ont un état de conscience altéré, par exemple des personnes atteintes de la maladie d'Alzheimer. Il est également possible que l'épisode d'incontinence survienne pendant le sommeil de l'individu. Des individus peuvent également être réticents à informer l'équipe soignante d'un épisode d'incontinence, lié par exemple à un sentiment de honte ou de gêne que peut ressentir la personne atteinte d'un tel trouble.

Ainsi, en raison du manque de personnel dans les EHPAD (23 postes de soignants pour 100 places en EHPAD, selon une étude menée par la Direction de Recherche, des Etudes de l'Evaluation et des Statistiques, publiée en juin 2018 dans Etudes & Résultats n°1067, https://drees.solidarites-sante.gouv.fr/IMG/pdf/er1067.pdf*),* la détection d'un épisode d'incontinence est susceptible de ne pas pouvoir être effectuée rapidement.

Aussi, pour faire face à un tel problème, il a été développé des dispositifs de détection de liquide dans un lit, permettant notamment d'alerter une tierce personne de la survenue d'un épisode d'incontinence. De cette manière, le soignant est instantanément informé d'une telle situation, et peut ainsi prendre en charge l'individu incontinent par un soin personnalisé.

Généralement, de tels capteurs comprennent deux électrodes disposées selon un motif prédéterminé, les électrodes n'étant pas reliées physiquement et électriquement entre elles. L'urine provoque une liaison électrique entre les électrodes, permettant la réalisation d'un circuit électrique fermé, autorisant le passage d'un courant. De tels capteurs sont généralement disposés au sein d'une protection telle qu'un coussin, destiné à recevoir l'urine.

US5537095 (Hill Rom) décrit un tel type de détection, le coussin étant lavable et comprenant une couche supérieure dans un matériau non tissé, une couche inférieure imperméable, la couche inférieure portant des bandes conductrices. Les bandes sont par exemple des bandes de mylar métallisé, en encre métallisée, ou formées d'un fil métallisé.

Les documents US 2668202 (Kaplan), US 2726294 (Health Guardian), US 3778570 (Shuman), WO 1998012997 (Oppenhejm), WO 2010124690 (Linak), US 2016374626 (Hill Rom), EP 3132780 (Hill Rom), et EP 3144668 (Medisens) décrivent des capteurs de liquide pour la détection de l'énurésie utilisant un tel principe.

Les sociétés Malem, et Astrid Medical mettent toutes deux sur le marché, respectivement sous l'appellation *"Malem Side Bedwetting Alarm"* et *"Astric Dry Bed"* un tapis souple réalisé dans un matériau thermoplastique destiné à être disposé sous les draps, pour détecter l'énurésie chez les enfants.

L'utilisation de tels dispositifs présente cependant des inconvénients.

La mise en place du capteur est contraignante, car nécessite un bon positionnement du coussin, en regard de l'appareil urinaire de l'individu incontinent. Or, une telle position peut varier selon la morphologie de l'individu incontinent. De surcroît, le coussin doit être maintenu en position afin de ne pas être déplacé volontairement ou involontairement par l'individu incontinent.

Par ailleurs, si l'individu incontinent transpire, le contact électrique pourrait être établi entre les électrodes, ce qui conduirait le capteur à détecter par erreur une incontinence urinaire. De plus, dans le cas où une des bandes, par exemple au niveau de la base des électrodes, est rompue, le circuit est susceptible de rester ouvert, malgré la présence d'urine. Par ailleurs, l'individu incontinent reste en contact avec des électrodes alimentées, ce qui en plus de nécessiter la mise en œuvre de précautions particulières pour éviter tout risque électrique, pourrait perturber les individus porteurs de stimulateurs cardiaques. Enfin, un tel détecteur n'est pas adapté pour la détection d'un épisode d'incontinence fécale.

Afin de répondre à une telle problématique, il a été proposé l'utilisation d'une encre conductrice directement sur une housse de matelas, comme présenté dans le document US 2007/0277323 (Bain, 2007). Néanmoins, les inconvénients liés à l'usage d'un capteur conductif ne sont pas résolus.

Le document FR 3062207 (Fogale Nanotech) propose un matelas muni de capteurs capacitifs aptes à détecter l'incontinence. Cependant, après être souillé par du liquide, le nettoyage d'un tel matelas est complexe donc couteux à mettre en œuvre.

L'invention se propose de résoudre les inconvénients de l'art antérieur.

Un premier objet de l'invention est de proposer un système de détection de l'incontinence d'un individu alité, permettant d'alerter une tierce personne sur la présence de liquide.

Un deuxième objet de l'invention est de proposer un tel système de détection qui soit fiable.

Un troisième objet de l'invention est de proposer un tel système de détection limitant le risque électrique chez l'individu alité.

Un quatrième objet de l'invention est de proposer un tel système de détection comprenant un drap-housse, offrant un ressenti de confort similaire à celui d'un drap-housse conventionnel.

Un cinquième objet de l'invention est de proposer un tel système de détection comprenant un drap-housse permettant l'utilisation de moyens de prévention des escarres.

Un sixième objet de l'invention est de proposer un tel système de détection comprenant un drap-housse lavable, de la même manière qu'un drap-housse conventionnel.

Un septième objet de l'invention est de proposer un lit comprenant un tel système de détection.

Un huitième objet de l'invention est de proposer une méthode de détection de l'incontinence d'une personne alitée, à l'aide du système de détection tel que présenté ci-dessus.

Un neuvième objet de l'invention est de proposer un produit programme d'ordinateur apte à mettre en œuvre la méthode présentée ci-dessus.

A cet effet, il est proposé, en premier lieu, un système de détection d'un épisode d'incontinence d'une personne alitée, comprenant un drap apte à recouvrir un matelas d'un lit, des capteurs capacitifs disposés sur le drap, les capteurs étant chacun apte à mesurer une longueur active correspondant à une longueur de capteur couverte par un fluide organique, une unité de traitement configurée pour collecter et analyser les longueurs mesurées par les capteurs, un capteur étant solidaire du drap.

Un tel système permet une détection efficace de l'incontinence d'une personne alitée, et délivre une alerte pour le cas où la personne est victime d'un épisode d'incontinence. Un tel système offre l'avantage de n'avoir d'impact défavorable ni sur le confort de la personne, ni sur les moyens de prévention (par exemple les dispositifs de prévention des escarres) et de soin apportés à l'individu occupant le lit.

Diverses caractéristiques supplémentaires peuvent être prévues, seules ou en combinaison :
- les capteurs sont disposés parallèlement entre eux, et transversalement sur le drap ;
- les capteurs présentent chacun la forme d'une bande ;
- les capteurs sont solidarisés au drap par tissage, impression ou collage ;
- les capteurs sont solidarisés au drap par l'un des moyens suivants : un fil, un pigment, une colle ;
- les capteurs comprennent une trame polyamide enduite d'argent ;
- une unité de traitement comprend un boitier muni d'un émetteur apte à autoriser la communication entre le système de détection et un terminal, par utilisation d'un protocole de communication filaire ou sans fil ;
- l'unité de traitement comprend une prise électrique femelle, et des moyens de fixation magnétiques ;
- le drap est un drap-housse.

Il est proposé, en deuxième lieu, un lit comprenant le système de détection tel que présenté ci-dessus.

Il est proposé, en troisième lieu, une méthode de détection d'un épisode d'incontinence d'une personne alitée sur un lit, telle que présenté ci-dessus, l'unité de traitement mettant en œuvre:
- une étape de relevé de longueurs actives de capteurs,
- une étape de comparaison avec des longueurs actives avec des longueurs de seuil prédéterminées,
- une étape de déclenchement d'une alerte, si les longueurs actives sont supérieures aux longueurs de seuil prédéterminées.

Il est proposé, en quatrième lieu, un produit programme d'ordinateur implémenté sur un support mémoire, susceptible d'être mis en œuvre sur un dispositif informatique et comprenant des instructions pour la commande de la méthode présentée ci-dessus.

D'autres caractéristiques et avantages de l'invention apparaîtront plus clairement et de manière concrète à la lecture de la description ci-après de modes de réalisation, laquelle est faite en référence aux dessins annexés dans lesquels :
- la figure 1 représente une vue schématique en perspective d'un lit muni d'un système de détection de l'incontinence d'une personne alitée,
- la figure 2 représente une vue schématique d'un drap-housse équipant un tel système de détection,
- la figure 3 est un graphique représentant des exemples de longueurs actives exprimées en mm, mesurées pour chaque capteur pour un individu alité, après un épisode d'incontinence,
- la figure 4 est un ordinogramme illustrant les étapes relatives à un mode de réalisation d'une méthode de détection de l'incontinence urinaire chez un individu alité.

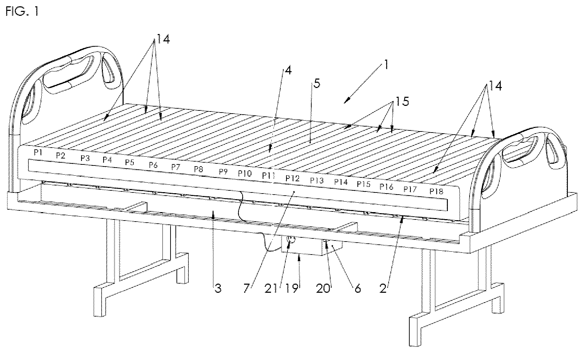

En se référant à la figure 1, il est représenté un lit **1** muni d'un matelas **2,** le lit **1** comprenant un système **3** de détection de l'incontinence chez un individu alité dans un tel lit **1.**

Le système **3** de détection comprend un drap **4,** muni de capteurs **5,** et une unité **6** de traitement, qui sont reliés l'un à l'autre, par exemple par l'intermédiaire d'un faisceau **7.**

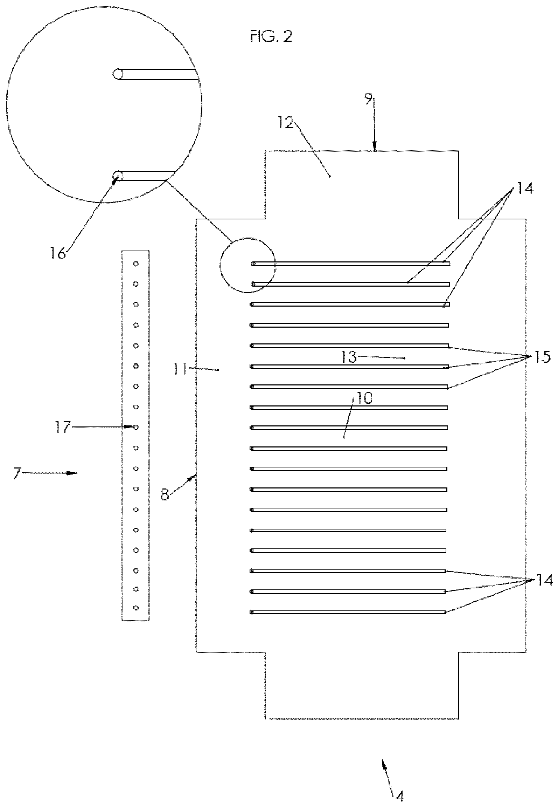

L'on se rapporte à la figure 2, représentant plus en détail le système **3** de détection.

Le drap **4** présente une silhouette sensiblement rectangulaire, comprenant un côté 8 longitudinal et un côté **9** transversal, le côté **8** longitudinal étant de longueur supérieure au côté **9** transversal.

Le drap **4** comprend une face **10** de contact, comprenant les capteurs **5** et une ou plusieurs faces **11** de fixation. La face **10** de contact est destinée à être positionnée sur une face supérieure du matelas **2** du lit **1,** tandis que les faces **11** de fixation s'étendent entre le bord du lit **1** et le matelas.

La face **10** de contact est destinée à être disposée entre l'individu et la face supérieure du matelas **2.** Ainsi, si l'individu alité subit un épisode d'incontinence, la face **10** de contact est recouverte partiellement d'urine ou de matière fécale.

Dans des modes de réalisation, un moyen élastique est placé en partie inférieure du drap **4,** formant ainsi un drap housse dont le bonnet est adapté au matelas.

Dans les modes de réalisation décrits, une housse de protection supplémentaire (non représentée sur les figures) recouvre le drap **4** du système **3** de détection, l'individu n'entrant pas directement en contact avec le drap **4** du système **3** de détection. La housse de protection comporte avantageusement une fermeture à glissière sur trois côtés, l'ouverture de la housse permettant l'accès aux capteurs pour le montage du faisceau **7.**

Une telle housse de protection limite les souillures du drap **4** lors d'épisode d'incontinence. Une telle housse de protection permet d'éviter un contact direct entre le drap **4** et l'individu, ce qui préserve la sensation de confort de l'individu. L'utilisation d'une telle housse de protection permet d'éviter le risque d'allergie que pourraient occasionner un contact prolongé entre les capteurs **5** et la peau de l'individu.

Dans le mode de réalisation représenté, les faces **11** de fixation sont agencées sur des rabats **12** rectangulaires, s'étendant sur chaque côté de la face **10** de contact. Le drap **4** ainsi mis en place se positionne de manière à ce que la face **10** de contact couvre bien le matelas **2.** Une telle mise en place est réalisable par toute personne, y compris par une personne ignorant l'existence de capteurs **5** au sein du drap **4.** L'utilisation du drap **4** est donc facilitée.

Dans d'autres modes de réalisation, non représentés, le drap **4** est un drap-housse comprenant une unique face **11** de fixation entourant la face **10** de contact et munie d'un lien élastique. Un tel drap-housse est facilement mis en place, et reste maintenu en position sur le matelas **2.** Par ailleurs, un tel drap-housse ne peut pas se déplacer si l'individu bouge.

Dans d'autres modes de réalisation, une sur-housse est formée par l'assemblage en un seul élément d'un drap **4** tel que représenté en figure 2 et d'une housse de protection. La housse de protection comporte avantageusement une fermeture à glissière, l'ouverture de la sur-housse permettant l'accès aux capteurs pour le montage du faisceau **7.**

Comme présenté ci-dessus, le drap **4** comprend une pluralité de capteurs **5,** préférentiellement disposé sur la face **10** de contact, qui est amenée à recevoir l'urine ou les excréments de la personne alitée.

Avantageusement, les capteurs **5** s'étendent également dans une ou plusieurs faces **11** de fixation, au moins partiellement, permettant de connecter le drap **4** avec le restant du système **3** de détection sans que l'individu alité soit gêné.

Les capteurs **5** sont ménagés avantageusement parallèlement entre eux, par exemple par l'intermédiaire de bandes **13, 14** rectilignes souples, qui s'étendent avantageusement de manière parallèles. Dans le mode de réalisation représenté, les bandes **13, 14** couvrent l'intégralité de la face **10** de contact.

Les capteurs **5,** qui dans le mode de réalisation représenté se matérialisent sous forme de bandes **13, 14** sont avantageusement disposés dans la largeur du drap **4,** c'est-à-dire dans une direction sensiblement parallèle au côté **9** transversal. Ainsi, lorsqu'un individu est allongé sur le lit **1,** les bandes **13, 14** sont disposées en regard de parties de corps. Des bandes **13** extrêmes positionnées à l'extrémité de la face **10** de contact sont situées en regard de la tête et des pieds de l'individu, tandis que les bandes **13, 14** centrales sont disposées au centre de la face **10** de contact, en regard et/ou à proximité de l'appareil urinaire et digestif de l'individu alité.

Les capteurs **5,** par exemple sous forme de bandes **13, 14** sont avantageusement disposés équidistants les uns des autres, permettant d'éviter d'avoir des parties du drap **4** non soumises à la détection. De cette façon, le drap **4** est utilisable pour toute morphologie de personne.

Les capteurs **5** sont des capteurs capacitifs, chacun des capteurs **5** définissant une première armature. Les capteurs **5** forment ensemble une surface **15** captante. Une deuxième armature est constituée du corps (corps de l'individu, urine ou excréments) recouvrant la surface **15** captante lors d'un épisode d'incontinence. Une capacité est ainsi crée entre les armatures.

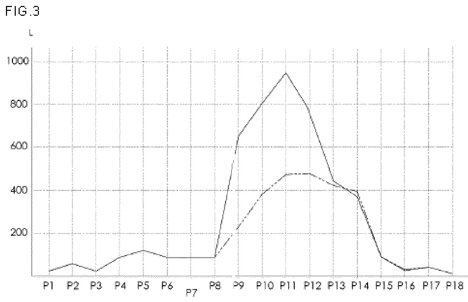

L'on se réfère à la figure 3 représentant les longueurs actives mesurées par chacun des capteurs **5.** Sans que cela soit limitatif, dix-huit capteurs **5** sont présents dans le drap, permettant la réalisation de dix-huit points de mesure **P1** à **P18,** le point de mesure **P1** étant à proximité de la tête de lit, tandis que le point de mesure **P18** est à proximité du pied de lit.

Lorsque l'individu est allongé sur le lit **1,** et n'est pas incontinent, une capacité nominale **Cn** est générée. Dans une telle situation, chacun des capteurs **5** mesure une longueur de bande nominale recouverte correspondant à une longueur de capteur **5** active nominale. Une telle longueur active **L** nominale correspond à la détection du corps de la personne alitée. Un exemple d'une telle situation est illustré par la courbe en trait mixte de la figure 3. Les valeurs mesurées par sept capteurs (P8 à P14) dépassent une valeur seuil de longueur active, par exemple égale à 50.

Lorsque survient un épisode d'incontinence, une capacité d'incontinence est générée. Une telle capacité d'incontinence est supérieure à la capacité nominale, en raison de la présence de capteurs **5** ayant une longueur **L** active accrue, par rapport à la longueur active en situation de non-incontinence. En effet, lors d'une incontinence, les bandes **14** centrales sont par exemple recouvertes d'une nappe de fluide, ou d'une protection absorbante saturée de fluide. Les capteurs **5** des bandes **13, 14** centrales sont alors davantage recouverts qu'en absence d'incontinence. Un exemple d'une telle situation est illustré par la courbe en trait plein de la figure 3. Les valeurs mesurées de sept capteurs (P8 à P14) dépassent une valeur seuil de longueur active, par exemple égale à 50, et sont significativement plus élevées que celles correspondant à l'occupation du lit par une personne en position allongée.

Dans le mode de réalisation représenté, le drap **4** comprend dix-huit bandes **13, 14** présentant une largeur de 90 mm, espacées entre elles de 100 mm. Dans d'autres modes de réalisation non représentés, le drap **4** comprend douze bandes **13, 14** présentant une largeur de 90 mm, et espacées les unes des autres de 145 mm. Un compromis est ainsi atteint entre le nombre de bandes **13, 14** et la qualité de détection des épisodes d'incontinence.

Dans un mode de réalisation, les capteurs **5** sont solidarisés au drap **4.** Les capteurs sont avantageusement des bandes **13, 14** conductrices, réalisé par exemple en tissus conducteur.

Les capteurs **5** sont par exemple collés, imprimés ou cousus sur le drap **4.**

Dans le mode de réalisation représenté, les capteurs **5** sont tissés au sein du drap **4.** De cette façon, le drap **4** présente une texture semblable à un drap conventionnel, c'est-à-dire sans capteur. Un individu alité ne perçoit donc pas la présence des capteurs 5. La résistance à l'usure des capteurs **5** est accrue par rapport à des capteurs imprimés, permettant une bonne tenue dans le temps.

Avantageusement, le drap **4** est réalisé dans un matériau suffisamment résistant pour pouvoir être nettoyé à haute température.

Avantageusement, les capteurs **5** comprennent des trames filaires réalisées à partir d'un matériau conducteur d'électricité, par exemple un fil polyamide enduit d'argent. Un tissu composé de telle fibre est facile à refendre pour couper en bandes et à coudre, ce qui facilite l'obtention du drap **4.** Un textile composé de tel fils offre une résistance accrue au lavage, ce qui n'impacte pas le traitement du linge dans l'institution ou l'établissement de soin. Enfin, un tissu réalisé avec de tels fils demeure souple, et n'est pas rugueux, ce qui est avantageux en terme de ressenti de confort pour l'individu alité, en particulier pour les personnes âgées qui ont une peau fragile.

Le drap **4** comprend par ailleurs des premiers points **16** de raccordement, disposés à une extrémité de chaque capteur **5,** afin de permettre le raccordement des capteurs **5** à l'unité **6** de traitement par l'intermédiaire du faisceau **7** sur des deuxièmes points **17** de raccordement complémentaires. Ainsi, lorsqu'un premier point **16** de raccordement est mâle, le deuxième point **17** de raccordement est femelle.

Dans le mode de réalisation représenté, les premiers points **16** de raccordement sont tous mâles et les deuxièmes points **17** de raccordement sont tous femelles, ce qui permet de faciliter la fabrication, et permet la réversibilité de connexion du faisceau **7.**

Dans d'autres modes de réalisation, non représentés, des premiers points **16** de raccordement sont mâles, les autres premiers points **16** de raccordement étant femelles. De manière complémentaire, des deuxièmes points **17** de raccordement sont femelles, et d'autres deuxièmes points **17** de raccordement sont mâles.

Avantageusement, les premiers points **16** de raccordement et les deuxièmes sont des éléments métalliques, s'engageant l'un dans l'autre par pression, par exemple des boutons pressions. De tels boutons offrent l'avantage d'être peu encombrant, permettant le positionnement des premiers points **16** de raccordement sur une face **11** de fixation. De tels boutons permettent un accrochage ou un décrochage facile, rapide et robuste du faisceau **7** sur le drap **4.** L'entretien du drap **4** est ainsi facilité.

Le faisceau **7** a pour objet de relier les bandes **13, 14** à l'unité **6** de traitement.

Le faisceau **7** englobe avantageusement un bandeau souple muni de deuxièmes points **17** de raccordement, qui sont connectés à l'unité **6** de traitement. La mise en place et le retrait du faisceau **7** est facile d'exécution. Le faisceau **7** est peu encombrant et ne gêne par conséquent pas l'individu. Le faisceau **7** peut être également recouvert par un drap supplémentaire, et est ainsi invisible pour l'individu alité.

Dans le mode de réalisation représenté, l'unité **6** de traitement est fixée sur le lit **1,** avantageusement sous le lit **1.**

L'unité **6** de traitement comprend les composants électroniques aptes à collecter, analyser et si besoin transmettre les données mesurées par les capteurs **5.**

L'unité **6** de traitement se présente avantageusement sous la forme d'un boitier **19,** permettant de protéger les composants électroniques.

L'unité **6** de traitement comprend des moyens **20** de fixation magnétiques, tels que des aimants permanents disposés sur le boitier **19,** permettant une fixation sur le cadre métallique du lit **1** réalisable rapidement, sans outil, et en ne requérant qu'un apprentissage limité. Ainsi, le système **3** de détection est amovible, ce qui peut être avantageux si l'individu est amené à changer de lit 1.

Avantageusement, l'unité 6 de traitement est fixée sous le lit 1, comme il peut être observé sur la figure 1. De cette manière, l'unité 6 de traitement n'est pas visible et accessible à l'individu alité.

De cette manière, l'unité 6 de traitement n'est pas à portée de main de l'individu. L'individu n'a donc pas la possibilité de manipuler l'unité 6 de traitement, ce qui est favorable notamment pour les personnes souffrant de pathologies psychiatriques. Le risque de dégradation du boitier suite à une manipulation inadéquate est éliminé.

Avantageusement, le boitier 19 présente une teinte identique ou similaire à la couleur de la structure métallique du lit. L'unité **6** de traitement est ainsi discrète, ce qui évite d'attirer l'attention de l'individu sur le boitier **19.**

Dans un mode de réalisation avantageux, afin de pallier au déficit de prises de courant dans les institutions, l'unité **6** de traitement comprend une prise électrique **21** femelle, disposée sur le boitier **19.**

L'unité **6** de traitement comprend par exemple un calculateur, un support mémoire, et un émetteur. Le calculateur permet notamment la collecte des données transmises par les capteurs **5,** et l'analyse de telles données. L'émetteur permet notamment la transmission de messages d'alerte via par exemple une interface vers un terminal.

Dans les modes de réalisation représentés, l'émetteur utilise un protocole de transmission sans fil, tel que le bluetooth, le wifi ou le réseau téléphonique mobile selon la norme 2G ou supérieure.

Dans d'autres modes de réalisation, non représentés, l'unité 6 de traitement est connectée par voie filaire au terminal, par exemple par l'intermédiaire d'un câble réseau.

Dans le mode de réalisation représenté, un terminal est un terminal fixe, par exemple un ordinateur, mais selon d'autres mises en œuvre, le terminal est un terminal mobile, tel qu'une tablette ou un smartphone. Un terminal mobile offre l'avantage de permettre une intervention rapide du personnel soignant ou d'une tierce personne aidante.

Dans des mises en œuvre préférentielles, l'unité **6** de traitement est connectée à plusieurs terminaux, qui peuvent être tous fixes, tous mobiles, ou à la fois mobiles et fixes.

L'unité **6** de traitement transmet des informations relatives à l'état du système **3** de détection, ainsi que des messages d'alarmes pour le cas où un épisode d'incontinence est détecté.

Avantageusement, un logiciel, par exemple une interface de visualisation installée sur un serveur dédié, permet la réception des messages, et la gestion du ou des systèmes **3** de détection installés. La gestion d'un parc de plusieurs lits **1,** chacun munis d'un système **3** de détection est ainsi facilitée pour le personnel soignant.

De manière à permettre une lecture efficace, l'interface permet par exemple un affichage des informations relatives à chaque système **3** de détection, par exemple par utilisation d'un système de couleurs. La gestion des épisodes d'incontinence est ainsi facilitée, un gain de temps pour le soignant est offert.

Avantageusement, l'interface permet un suivi des individus sur une période prédéterminée. Il est ainsi possible de déterminer à quel moment l'individu subit un épisode d'incontinence par suivi du système **3** de détection d'incontinence. En institution, le personnel peut ainsi individualiser de manière optimale la thérapie.

Dans un mode de réalisation préférentiel, l'unité **6** de traitement, soit directement, soit par exemple par l'intermédiaire de l'interface, envoie des messages d'alarmes sur des terminaux mobiles détenus par exemple par le personnel soignant. Ainsi, même lorsque l'intervenant n'a pas accès à l'interface, par exemple en cas d'intervention auprès d'un individu, il peut être informé de la présence d'un épisode d'incontinence chez un autre individu. De cette manière, le temps d'intervention pour prendre en charge l'individu est ainsi réduit.

Avantageusement, il est possible à l'aide du terminal mobile d'avoir accès à l'interface, ce qui permet d'avoir une vue globale du parc.

Ainsi, même si l'effectif du personnel est limité (par exemple la nuit), une personne peut intervenir rapidement sans devoir revenir vers le terminal fixe. L'intervenant optimise ainsi ses déplacements dans l'établissement, ce qui lui permet de passer plus de temps avec les individus alités et d'améliorer par exemple la prise en charge des individus incontinents.

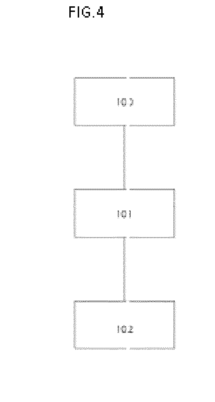

L'on se réfère à la figure 4, et l'on décrit un exemple de fonctionnement du système 3 de détection d'incontinence, et en particulier un procédé de détection d'un épisode d'incontinence à l'aide du système 3 de détection d'incontinence.

Dans une étape **100** de relevé, les capteurs **5** procèdent à une mesure de longueur **L** active de capteurs **5.** Les relevés sont effectués au cours d'une durée périodique prédéfinie.

Dans une étape **101** de comparaison, les longueurs **L** actives relevées sont comparées avec des longueurs de seuil prédéterminées, qui correspondent à un épisode d'incontinence.

Lorsqu'un nombre prédéfinis de capteurs **5** présentent une longueur **L** active supérieure à une longueur de seuil prédéterminée, un message d'alerte est envoyé à un terminal au cours d'une étape **102** de déclenchement.

Avantageusement, durant une telle étape **102** de déclenchement, l'unité **6** de traitement identifie le système **3** de détection qui détecte un épisode d'incontinence, avec un identifiant de lit. De cette manière, le message est envoyé aux terminaux accompagné de l'identifiant du lit, ce qui permet au soignant de savoir instantanément quel individu est victime d'un épisode d'incontinence.

Avantageusement, l'unité **6** de traitement envoie un message au terminal de sorte à permettre l'acquittement du message d'alerte.

Lorsqu'un nombre prédéfini de capteurs **5** présente une longueur **L** active inférieure à une longueur de seuil prédéterminée, alors aucune alerte n'est envoyée.

Le système **3** de détection offre de nombreux avantages, en particulier:
- une durée de vie maximisée, grâce aux capteurs **5** tissés sur le drap **4,**
- les capteurs ne sont pas perceptibles par la personne allongée sur le drap **4,** la sensation de confort de l'individu est préservée,
- une sureté d'utilisation, l'unité **6** de traitement étant mise à distance de l'individu,
- le système **3** de suivi n'empêche pas d'utiliser des moyens de prise en charge des escarres, par exemple des matelas spécifiques,
- une absence d'impact sur l'entretien, le drap **4** pouvant être nettoyé de la même manière que la literie non munie de capteurs **5,**
- une fabrication économique du drap **4** du fait de la possibilité d'utilisation de filaire pour la réalisation des capteurs **5.**

## Revendications

1. Système (3) de détection d'un épisode d'incontinence d'une personne alitée comprenant:
- un drap (4) apte à recouvrir un matelas (2) d'un lit (1),
- des capteurs (5) capacitifs sur le drap (4), les capteurs (5) étant chacun apte à mesurer une longueur active (L) correspondant à une longueur de capteur (5) couverte par un fluide organique,
- une unité (6) de traitement, configurée pour collecter et analyser les longueurs actives (L) mesurées par les capteurs (5),
le système (3) de détection étant **caractérisé en ce que** les capteurs (5) sont solidarisés au drap (4),
dans lequel, lorsqu'un individu est allongé sur le lit (1) et n'est pas incontinent, une capacité nominale (Cn) est générée, chacun des capteurs (5) étant configuré pour mesurer une longueur de bande nominale recouverte correspondant à une longueur de capteur (5) active nominale,
le système (3) de détection d'incontinence étant configuré pour fonctionner tel que :dans une étape (100) de relevé, les capteurs (5) procèdent à une mesure de longueur (L) active de capteurs (5), les relevés étant effectués au cours d'une durée périodique prédéfinie,
dans une étape (101) de comparaison, les longueurs (L) actives relevées sont comparées, avec des longueurs de seuil prédéterminées, qui correspondent à un épisode d'incontinence,
lorsqu'un nombre prédéfini de capteurs (5) présentent une longueur (L) active supérieure à une longueur de seuil prédéterminée, un message d'alerte est envoyé au cours d'une étape (102) de déclenchement.

2. Système (3) de détection selon la revendication 1, **caractérisé en ce qu'**un capteur (5) est solidarisé au drap (4) par l'un des moyens suivant :
- tissage,
- impression,
- collage.

3. Système (3) de détection selon la revendication 1 ou 2, **caractérisé en ce que** les capteurs (5) présentent chacun la forme d'une bande (13, 14).

4. Système (3) de détection selon l'une quelconque des revendications précédentes, **caractérisé en ce que** les capteurs (5) sont disposés parallèlement entre eux, et transversalement sur le drap (4).

5. Système (3) de détection selon l'une quelconque des revendications précédentes, **caractérisé en ce que** l'unité (6) de traitement comprend un boitier (19) muni d'un émetteur apte à autoriser la communication entre le système (3) de détection et un terminal, par utilisation d'un protocole de communication filaire ou sans fil.

6. Système (3) de détection selon l'une quelconque des revendications précédentes, **caractérisé en ce que** l'unité (6) de traitement comprend une prise électrique (21) femelle, et des moyens (20) de fixation magnétiques.

7. Système (3) de détection selon l'une quelconque des revendications précédentes, **caractérisé en ce que** le drap (4) est un drap-housse.

8. Lit (3) comprenant le système (3) de détection selon l'une quelconque des revendications précédentes.

9. Méthode de détection d'un épisode d'incontinence d'une personne alitée sur un lit selon la revendication précédente, l'unité de traitement mettant en œuvre:
• une étape de relevé de longueurs actives de capteurs,
• une étape de comparaison des longueurs actives avec des longueurs de seuil prédéterminées,
• une étape de déclenchement d'une alerte, si les longueurs actives sont supérieures aux longueurs de seuil prédéterminées.

10. Produit programme d'ordinateur implémenté sur un support mémoire, susceptible d'être mis en œuvre sur un dispositif informatique et comprenant des instructions pour la commande de d'une méthode selon la revendication précédente.

## Patentansprüche

1. System (3) zur Erkennung einer Inkontinenzepisode einer bettlägerigen Person, umfassend:
- ein Laken (4), das dazu ausgelegt ist, eine Matratze (2) eines Betts (1) zu bedecken,
- kapazitive Sensoren (5) auf dem Laken (4), wobei die Sensoren (5) jeweils dazu ausgelegt sind, eine aktive Länge (L) zu messen, die einer von einer organischen Flüssigkeit bedeckten Sensorlänge (5) entspricht,
- eine Verarbeitungseinheit (6), die so konfiguriert ist, dass sie die von den Sensoren (5) gemessenen aktiven Längen (L) erfasst und analysiert,
wobei das Erkennungssystem (3) **dadurch gekennzeichnet ist, dass** die Sensoren (5) fest mit dem Laken (4) verbunden sind,
wobei, wenn eine Person auf dem Bett (1) liegt und nicht inkontinent ist, eine Nennkapazität (Cn) erzeugt wird, wobei jeder der Sensoren (5) so konfiguriert ist, dass er eine Nennlänge eines abgedeckten Streifens misst, die einer aktiven Nennlänge des Sensors (5) entspricht,
wobei das Inkontinenzerkennungssystem (3) so konfiguriert ist, dass es wie folgt funktioniert: in einem Erhebungsschritt (100) nehmen die Sensoren (5) eine Messung der aktiven Länge (L) von Sensoren (5) vor, wobei die Erhebungen während einer vordefinierten Zeitdauer durchgeführt werden,
in einem Vergleichsschritt (101) werden die erhobenen aktiven Längen (L) mit vorbestimmten Schwellenlängen verglichen, die einer Inkontinenzepisode entsprechen,
wenn eine vordefinierte Anzahl von Sensoren (5) eine aktive Länge (L) aufweist, die größer ist als eine vordefinierte Schwellenlänge, wird während eines Auslöseschritts (102) eine Warnmeldung gesendet.

2. Erkennungssystem (3) nach Anspruch 1, **dadurch gekennzeichnet, dass** ein Sensor (5) durch eines der folgenden Mittel fest mit dem Laken (4) verbunden ist:
- Weben,
- Drucken,
- Verklebung.

3. Erkennungssystem (3) nach Anspruch 1 oder 2, **dadurch gekennzeichnet, dass** die Sensoren (5) jeweils die Form eines Streifens (13, 14) aufweisen.

4. Erkennungssystem (3) nach einem der vorhergehenden Ansprüche, **dadurch gekennzeichnet, dass** die Sensoren (5) parallel zueinander und quer auf dem Laken (4) angeordnet sind.

5. Erkennungssystem (3) nach einem der vorhergehenden Ansprüche, **dadurch gekennzeichnet, dass** die Verarbeitungseinheit (6) ein Gehäuse (19) umfasst, das mit einem Sender ausgestattet ist, der dazu ausgelegt ist, die Kommunikation zwischen dem Erkennungssystem (3) und einem Endgerät durch Verwendung eines drahtgebundenen oder drahtlosen Kommunikationsprotokolls zu ermöglichen.

6. Erkennungssystem (3) nach einem der vorhergehenden Ansprüche, **dadurch gekennzeichnet, dass** die Verarbeitungseinheit (6) eine elektrische Steckbuchse (21) und magnetische Befestigungsmittel (20) umfasst.

7. Erkennungssystem (3) nach einem der vorhergehenden Ansprüche, **dadurch gekennzeichnet, dass** das Laken (4) ein Spannbettlaken ist.

8. Bett (3), das das Erkennungssystem (3) nach einem der vorhergehenden Ansprüche umfasst.

9. Verfahren zur Erkennung einer Inkontinenzepisode einer bettlägerigen Person auf einem Bett nach dem vorhergehenden Anspruch, wobei die Behandlungseinheit Folgendes durchführt:
• einen Schritt des Erhebens der aktiven Sensorlängen,
• einen Schritt des Vergleichens der aktiven Längen mit vorbestimmten Schwellenlängen,
• einen Schritt des Auslösens einer Warnung, wenn die aktiven Längen größer sind als die vorbestimmten Schwellenlängen.

10. Computerprogrammprodukt, das auf einem Speichermedium implementiert ist, das auf einem Computergerät ausgeführt werden kann und Anweisungen zur Steuerung eines Verfahrens nach dem vorhergehenden Anspruch umfasst.

## Claims

1. A detection system (3) of an incontinence episode in a bedridden person, comprising:
- a sheet (4) capable of covering a mattress (2) of a bed (1),
- capacitive sensors (5) on the sheet (4), each of the sensors (5) being capable of measuring an active length (L) corresponding to a sensor length (5) covered by a bodily fluid,
- a processing unit (6), configured to collect and analyze the active lengths (L) measured by the sensors (5),
the detection system (3) being **characterized in that** the sensors (5) are secured to the sheet (4),
wherein, when an individual is lying on the bed (1) and is not incontinent, a nominal capacity (Cn) is generated, each of the sensors (5) being configured to measure a covered nominal strip length corresponding to a nominal active length of sensor (5),
the incontinence detection system (3) being configured to operate such that: in an acquisition step (100), the sensors (5) carry out a measurement of an active length (L) of sensors (5), the acquisitions being performed during a predefined periodic duration,
in a comparison step (101), the acquired active lengths (L) are compared, with predetermined threshold lengths, which correspond to an incontinence episode,
when a predefined number of sensors (5) have an active length (L) greater than a predetermined threshold length, an alert message is sent during a triggering step (102).

2. The detection system (3) according to claim 1, **characterized in that** a sensor (5) is secured to the sheet (4) by one of the following means:
- weaving,
- printing,
- bonding.

3. The detection system (3) according to claim 1 or 2, **characterized in that** each of the sensors (5) have the shape of a strip (13, 14).

4. The detection system (3) according to any one of the preceding claims, **characterized in that** the sensors (5) are disposed parallel to each other, and transversely on the sheet (4).

5. The detection system (3) according to any one of the preceding claims, **characterized in that** the processing unit (6) comprises a housing (19) provided with a transmitter capable of enabling communication between the detection system (3) and a terminal, using a wired or wireless communication protocol.

6. The detection system (3) according to any one of the preceding claims, **characterized in that** the processing unit (6) comprises a female electrical socket (21) and magnetic fastening means (20).

7. The detection system (3) according to any one of the preceding claims, **characterized in that** the sheet (4) is a fitted sheet.

8. A bed (3) comprising the detection system (3) according to any one of the preceding claims.

9. A method for detecting an incontinence episode in a bedridden person on a bed according to the preceding claim, the processing unit implementing:
• a step of acquiring active lengths of sensors,
• a step of comparing the active lengths with predetermined threshold lengths,
• a step of triggering an alert, if the active lengths are greater than the predetermined threshold lengths.

10. A computer program product implemented on a memory medium, likely to be implemented on a computer device and comprising instructions for controlling a method according to the preceding claim.
